Europäisches Patentamt

European Patent Office

Office européen des brevets

(11) Publication number: **0 143 341**
**A2**

# (12) EUROPEAN PATENT APPLICATION

(21) Application number: **84112915.8**

(22) Date of filing: **26.10.84**

(51) Int. Cl.⁴: **A 61 M 1/00**

(30) Priority: **29.11.83 SE 8306574**

(43) Date of publication of application: **05.06.85**
**Bulletin 85/23**

(84) Designated Contracting States: **BE CH DE FR GB IT LI LU NL**

(71) Applicant: **Gambro Lundia AB, Box 10101, S-220 10 Lund (SE)**

(72) Inventor: **Brorsson, Fritz Lennart, Jasminvägen 7, S-240 32 Flytinge (SE)**
Inventor: **Dahlberg, Bengt Ake Gustav, Masvägen 8 C, S-222 33 Lund (SE)**
Inventor: **Holmberg, Bengt Magnus, Studentvägen 10, S-237 00 Bjärred (SE)**

(74) Representative: **Boberg, Nils Gunnar Erik, Gambro AB Patent Department Box 10101, S-220 10 Lund (SE)**

(54) **Arrangement for the venting of a liquid.**

(57) An arrangement for the venting of a liquid which flows through a main duct (6), with the means (11–14) required for venting being arranged in a shunt line (15) connected in parallel and with means (12) for producing a recirculation of the liquid flowing through the shunt line (15) from a first point (16) in the main duct to a second point (17) arranged upstream of the same in the main duct with simultaneous venting.

The arrangement is intended in particular to be used in connection with medical treatment, e.g. for the venting of replacement liquid for haemofiltration or plasmafers or for venting of e.g. dialysis liquid.

0143341

## TITLE

ARRANGEMENT FOR THE VENTING OF A LIQUID

## TECHNICAL FIELD

The present invention relates to an arrangement for the venting of a liquid which flows through a main duct.

The arrangement is intended in particular to form part of a system for the feed of replacement liquid to a patient in connection with haemofiltration, plasmafers or similar treatment. It can also form part of a system for the preparation of such a liquid or other liquids which are to be vented, e.g. liquid intended to be used for dialysis.

The invention is thus intended first and foremost to be used in connection with different medical treatments. It will be clear, however, to those versed in the art that it can also be_ used more generally.

## BACKGROUND ART

In American patent specifications 4 158 034 and 4 293 409 for example a system is described for venting in connection with the preparation of fluid for dialysis. The venting is done here in a main duct in that the liquid is made to pass first through a throttling means, then a pump and finally a venting chamber, from the top of which accumulated air is separated. It is a disadvantage of this known system that the venting will depend to some extent on the existing flow conditions. If the flow changes rapidly for example there is a danger that a necessary adjustment of the venting would not be able to occur equally rapidly.

In the American patent specification 4 266 021 a different system of venting is described. Here the liquid which is to be vented can be conducted either via the venting arrangement or past the same in a parallel line. The venting will then take place either in that the air accumulated in a venting chamber is sucked off together with a part of the liquid or in that the air is entrained from the venting chamber via a liquid-tight but air-permeable membrane. The system described in this patent specification too is flow-dependent.


DISCLOSURE OF INVENTION

The abovementioned problem is solved by the present invention which relates to an arrangement of the aforementioned type, characterized in that the means required for venting are arranged in a shunt line connected in parallel and that a means is provided for producing recirculation of the liquid flow through the shunt line from a first point in the main duct to a second point arranged upstream of the same in the main duct with simultaneous venting.

In the main duct between the said first and second point a filter is preferably provided, e.g. a bacteria filter, in the manner as described for example in European patent application 81.103504.7 which has been published under number EP-A-O 042 939. In this way it is ensured that a bacteria-free liquid is produced which is suitable, for example, for dialysis, haemofiltration, plasmafers or similar treatment.

The said means for the venting preferably consist of a throttling means, a pump arranged downstream of it and a venting chamber arranged downstream of the pump. Through such a combination a very effective venting is obtained.

The inlet to the venting chamber is arranged appropriately at a higher level than the outlet. In this mannner the separation of discharged bubbles is facilitated, at the same time as the risk of these bubbles following along to the outlet instead of being separated is reduced. For the same reason an outlet is arranged at the highest point of the venting chamber for the air accumulated therein. This outlet may be constituted, for example, of a liquid-tight, but at the same time air-permeable, membrane which serves as a bacteria filter and prevents contamination of the liquid.

Between the throttling means and the pump suitable means are provided for the bringing about of an enhanced bubble formation. These may consist either of an expansion chamber arranged between the throttling means and the pump or of a lengthened duct between these parts so as to bring about
a corresponding expansion effect. Practical experiments have
shown in fact that it is not expedient to arrange the pump too
closely to the throttling means. The liquid requires to be
subjected to a partial vacuum during a period which must not
be too short before it is introduced into the pump so as to
ensure that the bubles effectively have time to be discharged.

As mentioned above, the invention is intended in the first
place to be applied in connection with a system for the feed of
replacement liquid to a patient in connection with haemofiltration,
plasmafers or similar treatment. The invention is described,
therefore, in the following with reference to just such a system
for haemofiltration.

BRIEF DESCRIPTION OF DRAWINGS

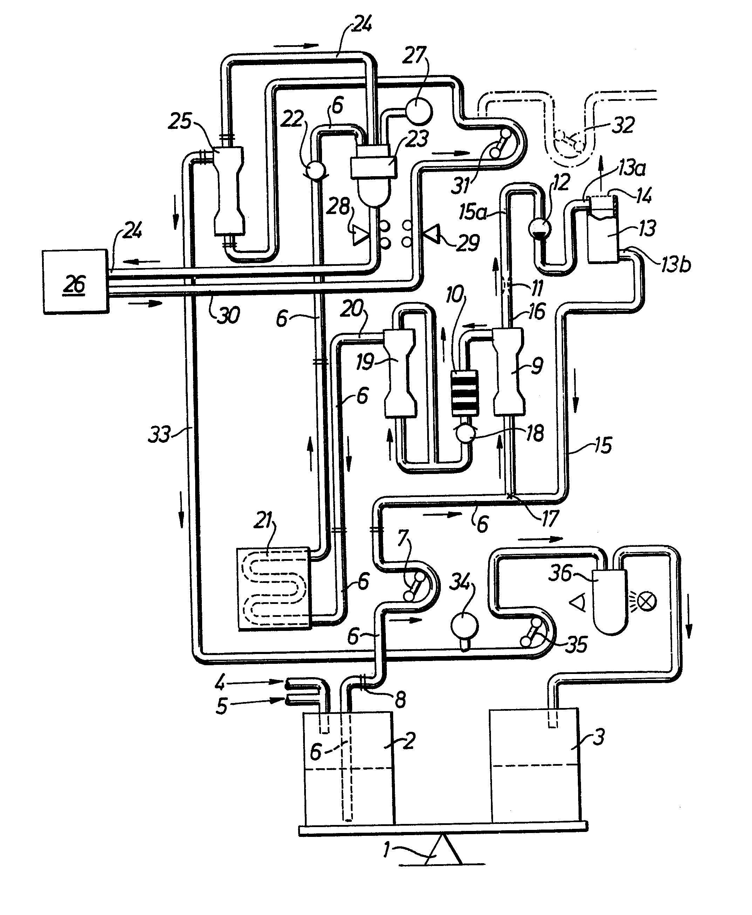

On the enclosed drawing a system is shown for the feed of
replacement liquid to a patient in connection with haemofiltration.

BEST MODE OF CARRYING OUT THE INVENTION

On the drawing is shown schematically a weighing system 1
with a vessel 2 intended for replacement liquid in the haemofiltration and a vessel 3 intended for liquid filtered off in the
haemofiltration. Numerals 4 and 5 respectively indicate
schematically imaginary inlets for water and concentrate respectively which in this example consequently are intended to be
mixed in the vessel 2. The mixed liquid is passed from the vessel
2 by means of a duct 6 with the help of a pump 7 via a connection
coupling 8. The liquid is conducted to a filter 9 and further
to a conductivity meter 10. A part of the liquid, however, is
conducted via a throttling means 11 with the help of a pump 12
to a venting chamber 13 whose inlet is designated 13a and the
outlet 13b. In the example shown this venting chamber is put
under pressure with the help of the pump 12 so that accumulated
air is pressed out through a membrane 14 which is liquid-tight
but air-permeable. Thus the venting is brought about in
a shunt line 15 arranged in relation to the main duct 6 which extends from a first point 16 in the main duct to a second point 17 in the main duct arranged upstream of this point. How great a part of the liquid is vented will depend on the ratio between the flow in the main duct 6 and the flow in the shunt line 15 and can be suitably selected by the appropriate dimensioning of the respective ducts or by the choice of setting of the operating characteristics of the throttling means 11 and of the pump 12 respectively. It is important in this connection that the pump 12 should not be placed too closely to the throttling means 11. On account of this an extra length of duct 15a has been inserted between these parts. Alternatively an expansion tank can be inserted here for the facilitating of the bubble formation. This must not be made too large, however, since in such a case the deflection of the conductivity meter will be delayed. The vented liquid is then conducted via the conductivity meter 10 and a nonreturn valve 18 to a second filter 19 which may be of the same type as the filter 9 or of some other more dense type. By delivering liquid to both ends of the filter 19 moreover good mixing conditions are ensured, at the same time as it is avoided that the filter includes any "dead point" with stagnant liquid.

The liquid is then conducted further through a duct 20 via heating device 21 and a nonreturn valve 22 to a drip chamber 23 arranged in the blood duct 24 from the haemofilter 25 to a patient 26. A pressure gauge is designated 27. Two shutoff valves 28,29 are provided in the blood duct 24 to the patient and the blood duct 30 from the patient respectively. Finally a blood pump and heparin pump are designated 31 and 32 respectively.

From the haemofilter 25 the filtered liquid is conducted via a duct 33 with a pressure gauge 34 by a pump 35 past a blood leakage detector 36 to the vessel 3 arranged on the weighing means 1.

Naturally the invention is not limited solely to the embodiment described above, but may be modified within the scope of the following claims. The schematically shown parts included in the system for example, can be modified substantially in respect of form as well as design. Reference is made, moreover, to Swedish patent application 8306573-0. which describes an alternative venting arrangement including parts which can be combined with the design according to the present invention.

CLAIMS

1. An arrangement for the venting of a liquid which flows through a main duct (6), c h a r a c t e r i z e d in that the means (11-14) required for venting are arranged in a shunt line (15) connected in parallel and that a means (12) is provided for producing recirculation of the liquid flow through the shunt line (15) from a first point (16) in the main duct (6) to a second point (17) arranged upstream of the same in the main duct with simultaneous venting.

2. An arrangement in accordance with claim 1, c h a r a c - t e r i z e d by a filter (9) provided between the said first and second points (16 and 17) in the main duct (6).

3. An arrangement in accordance with claim 1 or 2, c h a r a c t e r i z e d in that the said means (11-14) for venting consist of a throttling means (11), a pump (12) arranged downstream of it and a venting chamber (13-14) arranged downstream of the pump.

4. An arrangement in accordance with claim 3, c h a r a c - t e r i z e d in that the inlet (13a) to the venting chamber (13-14) is arranged at a higher level than the outlet (13b).

5. An arrangement in accordance with claim 3 or 4, c h a r a c t e r i z e d by an outlet (14) arranged at the highest point of the venting chamber (13-14) for the air accumulated therein, which preferably consists of a liquid-tight, but air-permeable, membrane.

6. An arrangement in accordance with anyone of claims 3-5, c h a r a c t e r i z e d by means (15a) arranged between the throttling means (11) and the pump (12) for the bringing about of an enhanced bubble formation.

7. An arrangement in accordance with claim 6, c h a r a c - t e r i z e d in that the said means (15a) for the bringing about of an enhanced bubble formation consists either of an expansion chamber arranged between the throttling means (11) and the pump (12) or of a lengthened duct (15a) between these parts for the bringing about of a corresponding expansion effect.

8.   An arrangement in accordance with anyone of the preceding claims, c h a r a c t e r i z e d in that it is included in a system for the feed of replacement liquid to a patient (26) in connection with haemofiltration, plasmafers or similar treatment.

0143341